(19) Europäisches Patentamt
European Patent Office
Office européen des brevets

(11) Numéro de publication : 0 396 474 B1

(12) # FASCICULE DE BREVET EUROPEEN

(45) Date de publication du fascicule du brevet : **10.03.93 Bulletin 93/10**

(51) Int. Cl.$^5$ : **A61K 31/135,** A61K 31/40, A61K 31/445, A61K 31/535

(21) Numéro de dépôt : **90401205.1**

(22) Date de dépôt : **04.05.90**

(54) **Utilisation de dérivés aminoalkoxy aromatiques pour le traitement des troubles du système cérébrovasculaire.**

(30) Priorité : **05.05.89 FR 8905988**

(43) Date de publication de la demande : **07.11.90 Bulletin 90/45**

(45) Mention de la délivrance du brevet : **10.03.93 Bulletin 93/10**

(84) Etats contractants désignés : **AT BE CH DE DK GB IT LI LU NL SE**

(56) Documents cités :
**BE-A- 895 464**
**W. FORTH et al.: "Allgemeine und Spezielle Pharmakologie und Toxikologie", Auflage 5, 1987, Seiten 278-284, Bi Wissenschaftsverlag, Mannheim, DE; "Vasodilatatoren in der Therapie der Herz- und Kreislauferkrankungen"**
**STROKE, vol. 21, no. 12, Dec. 90, IV-49-58**
**Calcium Entry Blockers in Cardiovascular and Cerebral Disfunctions, 1984; Martinus Nijhoff Publishers, pp.215**

(56) Documents cités :
**Nitrendipine, A. Scriabine et al., Urban & Schwarzenberg, Baltimore-Munich 1984, pp. 223-228**
**Annual Reports in Medicinal Chemistry, vol. 18, Academic Press, 1983, p. 82**

(73) Titulaire : **DELALANDE S.A.**
**32, rue Henri Regnault**
**F-92400 Courbevoie (FR)**

(72) Inventeur : **Pourrias, Bernard**
**6, Chemin du Chat Noir**
**F-91570 Bièvres (FR)**
Inventeur : **Ward, Mona, Résidence Gambetta**
**1-2, Square Henri Regnault**
**F-92400 Courbevoie (FR)**
Inventeur : **Santamaria, Raphael**
**15, Rue Henri Regnault**
**F-75014 Paris (FR)**

(74) Mandataire : **Kedinger, Jean-Paul et al**
**c/o Cabinet Malemont 42, avenue du Président Wilson**
**F-75116 Paris (FR)**

EP 0 396 474 B1

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention a pour objet l'utilisation de dérivés aromatiques comportant une chaîne aminoalkoxy et de leurs sels, pour le traitement des troubles du système cérébrovasculaire.

Plus précisément, lesdits dérivés répondent à la formule générale :

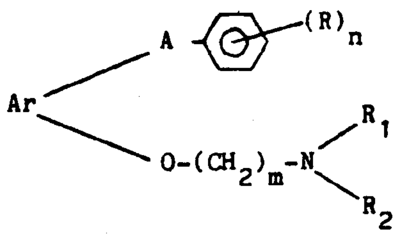

(I)

dans laquelle :

- R représente un atome d'hydrogène ou d'halogène, un groupe méthyle, hydroxyle, alkoxy où le reste alkyle comporte de 1 à 4 atomes de carbone, ou benzyloxy ;
- n prend la valeur 1 ou 2 quand R est différent de H ;
- m prend la valeur 2 ou 3 ;
- A représente un enchaînement ayant l'une quelconque des structures suivantes :

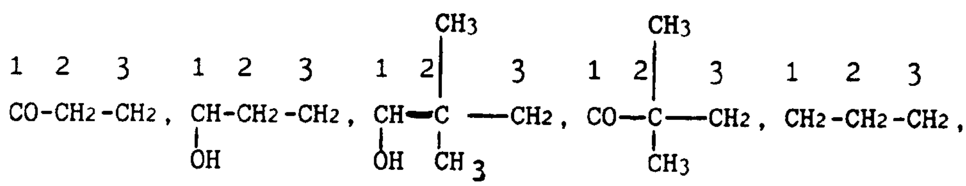

le groupe aromatique Ar étant relié à la position 1 de cet enchaînement,

- le couple ($R_1$, $R_2$) prend la valeur (H, alkyle en $C_1$-$C_4$), (H, cycloalkyle en $C_5$-$C_6$) ou (H, cycloalkylalkyle comportant de 4 à 8 atomes de carbone), excepté dans le cas où

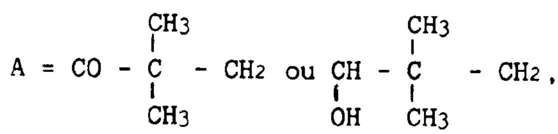

ou encore la valeur (alkyle en $C_1$-$C_4$, alkyle en $C_1$-$C_4$), $R_1$ et $R_2$ pouvant également former conjointement avec l'atome d'azote auquel ils sont liés, un radical choisi parmi les suivants : pyrrolidino, pipéridino, hexaméthylèneimino, morpholino ; et
- Ar représente :
  . soit un groupe benzènique de structure :

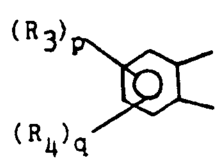

dans lequel $R_3$ représente un atome d'halogène ou un groupe nitro ou méthyle, $R_4$ = alkoxy de 1 à 4 atomes de carbone, p = 0, 1 ou 2, q = 0,1,2,3,4, p + q ≦ 4, avec les restrictions que lorsque A = CO-$CH_2$-$CH_2$ ou

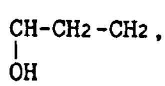

p et q ne peuvent prendre simultanément la valeur 0,

. soit un groupe naphtalènique ou benzodioxannique respectivement de structure :

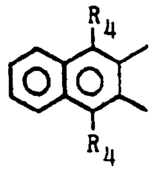
,
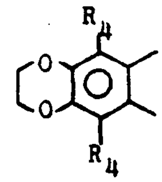

où $R_4$ a les mêmes significations que précédemment.

Quant aux sels des dérivés de formule (I), ils sont constitués par les sels d'addition d'acide minéral pharmaceutiquement acceptables, tels que les sels avec l'acide chlorhydrique et les sels d'addition d'acide organique pharmaceutiquement acceptables tels que les sels avec l'acide oxalique.

A titre d'exemples de dérivés de formule (I) et de leurs sels pharmaceutiquement acceptables, on pourra mentionner ceux répertoriés dans le Tableau I ci-après.

TABLEAU I

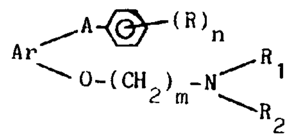 (I)

| No. de Code | -A - | $(R)_n$ | m | 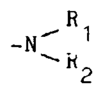 | 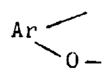 | Formule brute | Poids molé-culaire | Point de fusion (°C) | Forme | ANALYSE ELEMENTAIRE OU SPECTRE R.M.N. | | | |
|---|---|---|---|---|---|---|---|---|---|---|---|---|---|
| | | | | | | | | | | % | C | H | N |
| 1 | $-CO-CH_2-CH_2-$ | 4-OH | 2 | 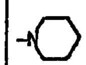 | 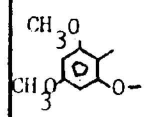 | $C_{24}H_{31}NO_5$ | 413,49 | 88 | Base | RMN ($CDCl_3$) δ ppm=6,82,m et 6,10,s (OH aromatiques);4,1,t ($OCH_2$);3,72 et 3,8,s ($2CH_3O$);2,4 à 3,m (10 H: $OCH_2CH_2$ et $CH_2-N<(CH_2)(CH_2)$);1,5,m ($CH_2CH_2CH_2$) | | | |
| 2 | $-CO-CH_2-CH_2-$ | " | " | " | 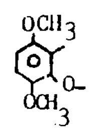 | $C_{24}H_{32}ClNO_5$ | 449,96 | 181 | HCl | Cal. | 64,06 | 7,17 | 3,11 |
| | | | | | | | | | | Tr. | 63,98 | 7,09 | 3,08 |
| 3 | 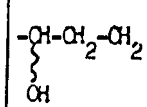 | H | " | " | " | $C_{24}H_{33}NO_4$ | 399,51 | 79 | Base | Cal. | 72,15 | 8,33 | 3,51 |
| | | | | | | | | | | Tr. | 72,04 | 8,51 | 3,45 |
| 4 | " | 4-F | " | " | " | $C_{24}H_{32}FNO_4$ | 417,50 | Huile | " | Cal. | 69,04 | 7,73 | 3,36 |
| | | | | | | | | | | Tr. | 68,49 | 7,56 | 3,15 |

TABLEAU I (suite)

| No. de Code | -A- | $(R)_n$ | m | 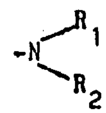 | 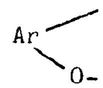 | Formule brute | Poids moléculaire | Point de fusion (°C) | Forme | ANALYSE ELEMENTAIRE OU SPECTRE R.M.N. % | C | H | N |
|---|---|---|---|---|---|---|---|---|---|---|---|---|---|
| 5 | $-CH-CH_2-CH_2-$<br>OH | 4-Cl | 2 | 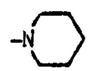 | 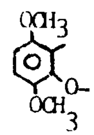 | $C_{24}H_{32}ClNO_4$ | 433,96 | Huile | Base | Cal. | 66,42 | 7,43 | 3,23 |
| | | | | | | | | | | Tr. | 65,80 | 7,95 | 3,34 |
| 6 | " | $4-CH_3$ | " | " | " | $C_{25}H_{35}NO_4$ | 413,54 | " | " | Cal. | 72,61 | 8,53 | 3,39 |
| | | | | | | | | | | Tr. | 72,31 | 8,58 | 3,27 |
| 7 | " | $4-CH_3O$ | " | " | " | $C_{25}H_{35}NO_5$ | 429,54 | " | " | Cal. | 69,90 | 8,21 | 3,26 |
| | | | | | | | | | | Tr. | 69,25 | 8,33 | 3,14 |
| 8 | " | 2-OH | " | " | " | $C_{24}H_{34}ClNO_5$ + 4 % $H_2O$ | 470,81 | 88 | HCl + 4% $H_2O$ | Cal. | 61,22 | 7,73 | 2,98 |
| | | | | | | | | | | Tr. | 61,50 | 7,93 | 2,97 |

TABLEAU I (suite)

| No. de Code | -A - | $(R)_n$ | m | 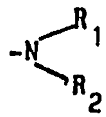 | 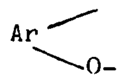 | Formule brute | Poids moléculaire | Point de fusion (°C) | Forme | ANALYSE ELEMENTAIRE OU SPECTRE R.M.N. % | C | H | N |
|---|---|---|---|---|---|---|---|---|---|---|---|---|---|
| 9 | 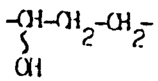 | 3-OH | 2 | 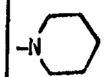 | 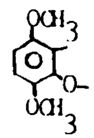 | $C_{24}H_{33}NO_5$ | 415,52 | 128 | Base | Cal. | 69,31 | 8,01 | 3,37 |
| | | | | | | | | | | Tr. | 69,25 | 8,02 | 3,19 |
| 10 | " | 4-OH | " | " | " | $C_{24}H_{33}NO_5$ | 415,52 | 142 | " | Cal. | 69,31 | 8,01 | 3,37 |
| | | | | | | | | | | Tr. | 69,29 | 8,10 | 3,21 |
| 11 | " | " | 3 | " | " | $C_{25}H_{35}NO_5$ | 429,54 | 118 | " | Cal. | 69,90 | 8,21 | 3,26 |
| | | | | | | | | | | Tr. | 69,95 | 8,35 | 3,30 |
| 12 | " | " | 2 | 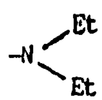 | " | $C_{23}H_{33}NO_5$ | 403,50 | 90 | " | Cal. | 68,46 | 8,24 | 3,47 |
| | | | | | | | | | | Tr. | 68,28 | 8,39 | 3,46 |

TABLEAU I (suite)

| No. de Code | -A- | $(R)_n$ | m | 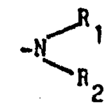 | 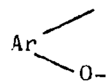 | Formule brute | Poids moléculaire | Point de fusion (°C) | Forme | ANALYSE ELEMENTAIRE OU SPECTRE R.M.N. % | C | H | N |
|---|---|---|---|---|---|---|---|---|---|---|---|---|---|
| 13 | $-\underset{OH}{CH}-CH_2-CH_2-$ | 4-OH | 2 | 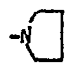 | 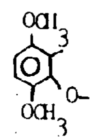 | $C_{23}H_{31}NO_5$ | 401,49 | 159 | Base | Cal. | 68,80 | 7,78 | 3,49 |
| | | | | | | | | | | Tr. | 68,14 | 7,84 | 3,52 |
| 14 | " | " | " | 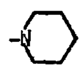 | 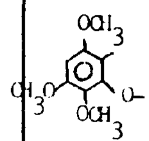 | $C_{25}H_{35}NO_6$ + 3/4 HOOC-COOH | 513,06 | 175 | 3/4 oxalate | Cal. | 62,03 | 7,17 | 2,73 |
| | | | | | | | | | | Tr. | 61,89 | 7,32 | 2,73 |
| 15 | " | " | " | " | 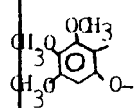 | $C_{25}H_{35}NO_6$ | 445,54 | 117 | Base | Cal. | 67,39 | 7,92 | 3,14 |
| | | | | | | | | | | Tr. | 67,16 | 8,08 | 2,89 |
| 16 | " | " | " | " | 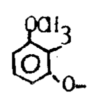 | $C_{24}H_{32}NO_6$ + 1,2 % $H_2O$ | 435,56 | 148 | 0,5 oxalate +1,2 % $H_2O$ | Cal. | 66,17 | 8,00 | 3,22 |
| | | | | | | | | | | Tr. | 66,32 | 7,62 | 3,17 |

TABLEAU I (suite)

| No. de Code | -A- | (R)$_n$ | m | 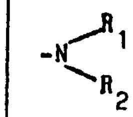 | 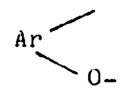 | Formule brute | Poids molé-culaire | Point de fusion (°C) | Forme | ANALYSE ELEMENTAIRE OU SPECTRE R.M.N. | | | |
|---|---|---|---|---|---|---|---|---|---|---|---|---|---|
| | | | | | | | | | | % | C | H | N |
| 17 | 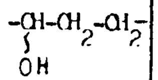 | 4-OH | 2 | 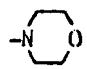 | 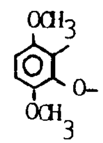 | $C_{23}H_{31}NO_6$ | 417,49 | 107 | Base | Cal. | 66,17 | 7,48 | 3,36 |
| | | | | | | | | | | Tr. | 65,88 | 7,70 | 3,47 |
| 18 | " | " | " | 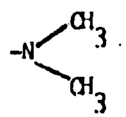 | " | $C_{21}H_{29}NO_5$ + 1,1 % $H_2O$ | 379,70 | 105 | Base + 1,1 % $H_2O$ | Cal. | 66,43 | 7,83 | 3,69 |
| | | | | | | | | | | Tr. | 66,03 | 7,98 | 3,86 |
| 19 | " | " | " | 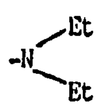 | 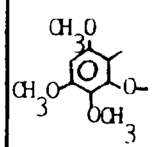 | $C_{24}H_{35}NO_6$ | 433,53 | 112 | Base | Cal. | 66,49 | 8,14 | 3,23 |
| | | | | | | | | | | Tr. | 66,40 | 8,48 | 2,93 |
| 20 | " | " | " | " | 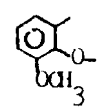 | $C_{25}H_{33}NO_8$ + 1,7 % $H_2O$ | 483,75 | 103 | Oxalate +1,7 % $H_2O$ | Cal. | 62,07 | 7,07 | 2,90 |
| | | | | | | | | | | Tr. | 62,24 | 7,14 | 3,04 |

TABLEAU I (suite)

| No. de Code | -A- | (R)$_n$ | m | 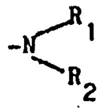 | 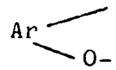 | Formule brute | Poids molé-culaire | Point de fusion (°C) | Forme | ANALYSE ELEMENTAIRE OU SPECTRE R.M.N. | | | |
|---|---|---|---|---|---|---|---|---|---|---|---|---|---|
| | | | | | | | | | | % | C | H | N |
| 21 | $-CH(OH)-CH_2-CH_2-$ | 4-O⌒Ø | 2 | 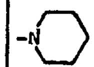 | 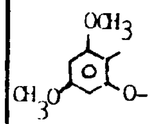 | $C_{31}H_{39}NO_5$ | 505,63 | Huile | Oxalate + 1,7 % $H_2O$ | RMN ($CDCl_3$) δ ppm=6,8 à 7,5,m (9 H aromatiques);6,25,s (2 H aromatiques en 3 et 5);5,05,s ($OCH_2$-Ø); 5,1,m (CH-O);4,1,m (OH et $OCH_2$); 3,8,s ($2CH_3O$);2 à 2,8 m (10 H : $-CH_2-CH_2$ et $CH_2-N(CH_2)_2$ );1,4 à 1,8 m (-N(CH$_2$)$_2$ CH$_2$). | | | |
| 22 | $-CH(OH)-CH_2-CH_2-$ | 3,4-di O⌒Ø | " | " | 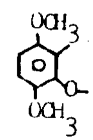 | $C_{38}H_{45}NO_6$ | 611,75 | " | Base | RMN ($CDCl_3$) δ ppm=7,2 à 7,6,m (10 H benzyliques);6,6 à 6,9,m (5 H aromatiques) ; 5,1,s ($2OCH_2$Ø) ; 5 à 5,3, m (OH et CH-O) ; 4,4 et 3,4,m ($OCH_2$) ; 3,7 et 3,78, s ($2CH_3O$) ; 2,2 à 2,6, m et 1,4 à 1,8, m ($CH_2CH_2$ et $CH_2$-N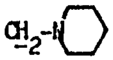 ) | | | |
| 23 | " | 3,4-diOH | " | " | " | $C_{24}H_{34}ClNO_6$ + 4,8 % $H_2O$ | 491,57 | 140 | HCl + 4,8 % $H_2O$ | Cal. | 58,63 | 7,51 | 2,85 |
| | | | | | | | | | | Tr. | 58,66 | 7,42 | 2,67 |

TABLEAU I (suite)

| Numéro de Code | 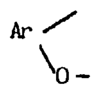 | - A - | $(R)_n$ | m | 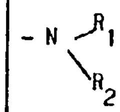 | Forme | Formule brute | Poids molécu-laire | Point de fusion (° C) | ANALYSE ELEMENTAIRE OU SPECTRE RMN % | C | H | N |
|---|---|---|---|---|---|---|---|---|---|---|---|---|---|
| 60 | 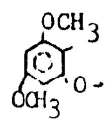 | $-(CH_2)_3-$ | 4-OH | 2 | 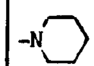 | Base | $C_{24}H_{33}NO_4$ | 399,51 | 136 | Cal. | 72,15 | 8,33 | 3,51 |
| | | | | | | | | | | Tr. | 71,94 | 8,44 | 3,38 |
| 61 | 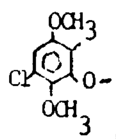 | $-CO-CH_2-CH_2-$ | " | " | " | " | $C_{24}H_{30}ClNO_5$ | 447,94 | 120 | Cal. | 64,35 | 6,75 | 3,13 |
| | | | | | | | | | | Tr. | 64,22 | 6,96 | 2,94 |
| 62 | 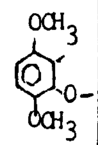 | $-\underset{OH}{CH}-CH_2-CH_2-$ | " | " | 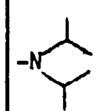 | " | $C_{25}H_{37}NO_5$ | 431,55 | 75 | Cal. | 69,57 | 8,64 | 3,25 |
| | | | | | | | | | | Tr. | 69,19 | 8,82 | 3,51 |
| 63 | 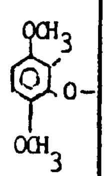 | " | " | " | 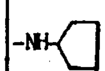 | Oxalate | $C_{26}H_{35}NO_9$ | 505,55 | 197 | Cal. | 61,77 | 6,98 | 2,77 |
| | | | | | | | | | | Tr. | 61,52 | 7,36 | 2,88 |

TABLEAU I (Suite)

| Numéro de Code | 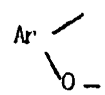 | - A - | $(R)_n$ | m | 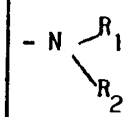 | Forme | Formule brute | Poids moléculaire | Point de fusion (° C) | ANALYSE ELEMENTAIRE OU SPECTRE RMN % | C | H | N |
|---|---|---|---|---|---|---|---|---|---|---|---|---|---|
| 64 | 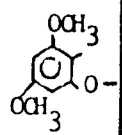 | 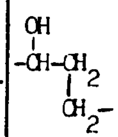 | 4-OH | 2 | 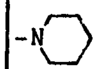 | Base | $C_{24}H_{33}NO_5$ | 415,51 | 128 | Cal. | 69,37 | 8,01 | 3,37 |
| | | | | | | | | | | Tr. | 68,86 | 8,00 | 3,36 |
| 65 | 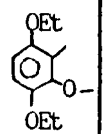 | " | " | " | 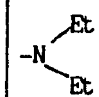 | " | $C_{25}H_{37}NO_5$ | 431,55 | 80 | Cal. | 69,57 | 8,64 | 3,25 |
| | | | | | | | | | | Tr. | 69,07 | 8,77 | 3,44 |
| 66 | 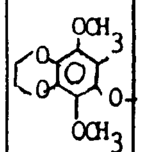 | " | " | " | 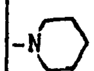 | Base | $C_{26}H_{35}NO_7$ | 473,55 | 158 | Cal. | 65,94 | 7,45 | 2,96 |
| | | | | | | | | | | Tr. | 65,63 | 7,39 | 2,82 |
| 67 | 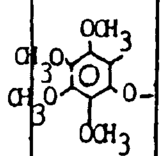 | 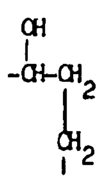 | " | 2 | 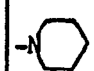 | Chlorhydrate | $C_{26}H_{38}ClNO_7$ | 512,03 | 105 | Cal. | 60,98 | 7,48 | 2,74 |
| | | | | | | | | | | Tr. | 60,65 | 7,79 | 2,91 |

TABLEAU I (Suite)

| Numéro de Code | Ar / O – | - A - | $(R)_n$ | m | - N R1 R2 | Forme | Formule brute | Poids moléculaire | Point de fusion (° C) | ANALYSE ELEMENTAIRE OU SPECTRE RMN % | C | H | N |
|---|---|---|---|---|---|---|---|---|---|---|---|---|---|
| | 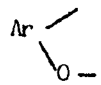 | | | | 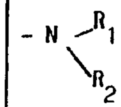 | | | | | | | | |
| 68 | 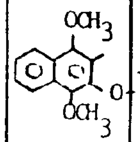 | 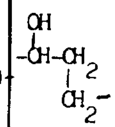 | 4-OH | 2 | 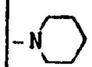 | Base + 0,8% $H_2O$ | $C_{28}H_{35}NO_5$ + 0,8 % $H_2O$ | 469,32 | 180 | Cal. | 71,65 | 7,61 | 2,99 |
| | | | | | | | | | | Tr. | 71,47 | 7,84 | 2,91 |
| 69 | 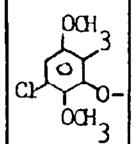 | " | " | " | " | Base | $C_{24}H_{32}ClNO_5$ | 449,96 | 116 | (Oxalate) Cal. | 57,83 | 6,35 | 2,59 |
| | | | | | | Oxalate | $C_{26}H_{34}ClNO_9$ | 539,99 | 131 | Tr. | 57,67 | 6,47 | 2,88 |
| 70 | " | " | " | " | 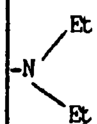 | Base | $C_{23}H_{32}ClNO_5$ | 437,95 | 124 | Cal. | 63,07 | 7,37 | 3,20 |
| | | | | | | | | | | Tr. | 62,80 | 7,46 | 3,23 |
| 71 | " | " | " | " | 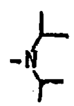 | " | $C_{25}H_{36}ClNO_5$ | 466,00 | 102 | Cal. | 64,43 | 7,79 | 3,01 |
| | | | | | | | | | | Tr. | 64,51 | 8,05 | 2,98 |

TABLEAU I (Suite)

| Numéro de Code | 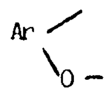 | - A - | $(R)_n$ | m | 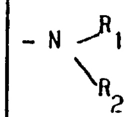 | Forme | Formule brute | Poids moléculaire | Point de fusion (° C) | ANALYSE ELEMENTAIRE OU SPECTRE RMN % | C | H | N |
|---|---|---|---|---|---|---|---|---|---|---|---|---|---|
| 72 | 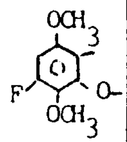 | 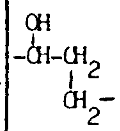 | 4-OH | 2 | 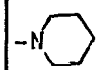 | Base | $C_{24}H_{32}FNO_5$ | 433,50 | 122 | Cal. | 66,49 | 7,44 | 3,23 |
| | | | | | | | | | | Tr. | 66,43 | 7,53 | 3,01 |
| 73 | 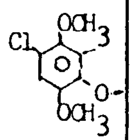 | " | " | " | " | " | $C_{24}H_{32}ClNO_5$ | 449,96 | 132 | Cal. | 64,06 | 7,17 | 3,11 |
| | | | | | | | | | | Tr. | 63,95 | 6,89 | 2,83 |
| 74 | 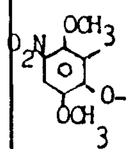 | " | " | " | " | " | $C_{24}H_{32}N_2O_7$ | 460,51 | 162 | Cal. | 62,59 | 7,00 | 6,08 |
| | | | | | | | | | | Tr. | 62,84 | 7,40 | 5,99 |
| 75 | 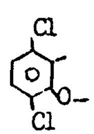 | 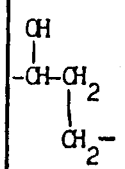 | " | 2 | 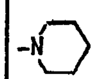 | Base | $C_{22}H_{27}Cl_2NO_3$ | 424,36 | 147 | Cal. | 62,26 | 6,41 | 3,30 |
| | | | | | | | | | | Tr. | 62,41 | 6,60 | 3,25 |

TABLEAU I (Suite)

| Numéro de Code | Ar / O– | - A - | (R)n | m | - N R1 R2 | Forme | Formule brute | Poids moléculaire | Point de fusion (° C) | ANALYSE ELEMENTAIRE OU SPECTRE RMN % | C | H | N |
|---|---|---|---|---|---|---|---|---|---|---|---|---|---|
| | 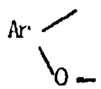 | | | | 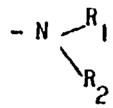 | | | | | | | | |
| 76 | 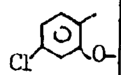 | 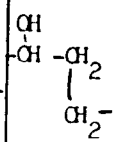 | 4-OH | 2 | 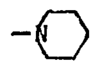 | Base | $C_{22}H_{28}ClNO_3$ | 389,91 | 153 | Cal. | 67,76 | 7,24 | 3,59 |
| | | | | | | | | | | Tr. | 67,74 | 7,41 | 3,58 |
| 77 | 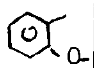 | 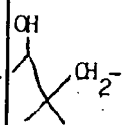 | " | " | " | HCl | $C_{24}H_{34}ClNO_3$ | 419,98 | 210 | Cal. | 68,63 | 8,16 | 3,34 |
| | | | | | | | | | | Tr. | 68,72 | 8,10 | 3,32 |
| 78 | 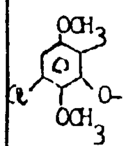 | 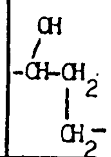 | H | " | " | base | $C_{24}H_{32}ClNO_4$ | 433,96 | 89 | Cal. | 66,42 | 7,43 | 3,23 |
| | | | | | | | | | | Tr. | 66,50 | 7,51 | 3,29 |
| 79 | " | $-(CH_2)_3-$ | 4-OH | " | " | Oxalate | $C_{26}H_{34}ClNO_8$ | 523,99 | 164 | Cal. | 59,59 | 6,54 | 2,67 |
| | | | | | | | | | | Tr. | 59,73 | 6,43 | 2,96 |

TABLEAU I (Suite)

| Numéro de Code | Ar 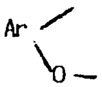 | - A - | $(R)_n$ | m | 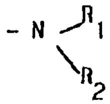 | Forme | Formule brute | Poids molécu-laire | Point de fusion (° C) | ANALYSE ELEMENTAIRE OU SPECTRE RMN |
|---|---|---|---|---|---|---|---|---|---|---|
| 80 | 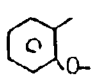 | 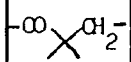 | 4-$OCH_2$Ø | 2 | 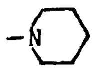 | Base | $C_{31}H_{37}NO_3$ | 471,61 | < 50 | RMN:($CDCl_3$) δ ppm=6,6 à 7,4,m(13H aromatiques);5,0,s($CH_2$-Ø);4,0,t et 2,6,t ($O-CH_2-CH_2$);2,85,s($CH_2$);2,4,m(4 H Pipéridiniques);1,4,m(6H pipéridiniques);1,15,s(2 $CH_3$). |
| 81 | " | " | 4-OH | " | " | " | $C_{24}H_{31}NO_3$ | 381,49 | Huile | RMN($CDCl_3$) δ ppm=7,4,m(OH);6,6 à 7,2, m (8H aromatiques);4,05 et 2,6,t ($O-CH_2-CH_2$);2,8,s($CH_2$);1,15,s (2 $CH_3$); 2,5 et 1,55,m(10 H pipéridiniques). |
| 82 | 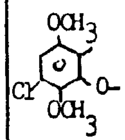 | 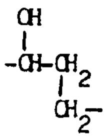 | 4-$OCH_2$Ø | " | " | " | $C_{31}H_{38}ClNO_5$ | 541,08 | " | RMN($CDCl_3$) δ ppm=7,4,s(5 H benzyliques);6,5 à 7,2,m (5 H aromatiques);5,s($OCH_2$);5,05, m(OH);4,2,m(C-H/-OH);3,65et 3,75,s($CH_3O$);3,7 et 2,7,m $OCH_2-CH_2$);2,4 et 1,5,m(10H pipéridiniques) |
| 83 | 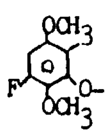 | " | " | " | " | " | $C_{31}H_{38}FNO_5$ | 523,62 | " | RMN ($CDCl_3$) δ ppm=7,4,s(5H benzyliques); 6,8,d et 7,10,d(4H aromatiques);6,4,d (J H-F) (1 H aromatique); 5,s ($CH_2$) ; 5,05, m (CH-O);4,5,4,0 et 2,8, m ($OCH_2CH_2$-); 3,65 et 3,8,s ($CH_3O$) ; 2,4 et 1,6,m (10 H pipéridiniques) |

TABLEAU I (Suite)

| Numéro de Code | 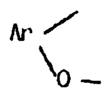 | - A - | (R)n | m | 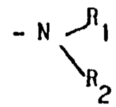 | Forme | Formule brute | Poids moléculaire | Point de fusion (° C) | ANALYSE ELEMENTAIRE OU SPECTRE RMN |
|---|---|---|---|---|---|---|---|---|---|---|
| 84 | 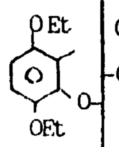 | 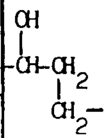 | 4OCH$_2$Ø | 2 | 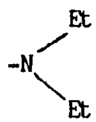 | Base | $C_{32}H_{43}NO_5$ | 521,67 | Huile | RMN($CDCl_3$) δ ppm=7,35,s(5 H benzyliques);6,4 à 7,4,m(6H aromatiques) 5,35,m(OH);5,s($CH_2$);4,45,m(CH-O); 3,6 à 4,10,m (3 $OCH_2$);2,4 à 2,8,m (-$CH_2$-$CH_2$-et N ($CH_2$)$_3$: 10 protons); 0,9 à 1,4,m,(12 H : 4 $CH_3$). |
| 85 | 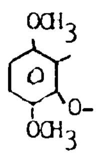 | " | " | " | 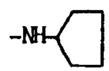 | " | $C_{31}H_{39}NO_5$ | 505,63 | Huile | RMN($CDCl_3$), δ ppm=7,35,s(5H benzyliques);6,5 à 7,4,m(6H aromatiques); 5.,s($CH_2$)et 5,05,m(CH-O);3,65 et 3,75, s($OCH_3$);4,10,m et 1,4 à 3,4,m(18 protons). |
| 86 | 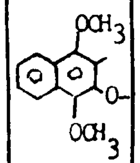 | " | " | 2 | 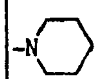 | " | $C_{35}H_{41}NO_5$ | 555,69 | Huile | RMN : ($CDCl_3$) δ ppm = 8,set 6,7 à 7,4(13H aromatiques);5,s($CH_2$);3,85,s (2$OCH_3$);4,5,m et 1,4 à 2,8,m (19 protons). |
| 87 | 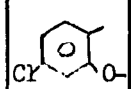 | 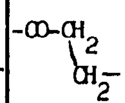 | 4-OH | " | " | " | $C_{22}H_{26}ClNO_3$ | 387,89 | 148 | RMN($CDCl_3$) δ ppm=9,3,m(OH);6,6 à 7,6, m(7H aromatiques);4,2 et 2,6,t($OCH_2$-$CH_2$-);3,25et 2,7,t(CO-$CH_2CH_2$);2,4 et 1,5,m (10 H pipéridiniques). |

TABLEAU I (Suite)

| Numéro de Code | 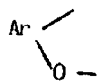 | - A - | (R)n | m | 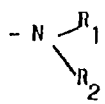 | Forme | Formule brute | Poids moléculaire | Point de fusion (° C) | ANALYSE ELEMENTAIRE OU SPECTRE RMN |
|---|---|---|---|---|---|---|---|---|---|---|
| 88 | 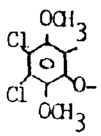 | 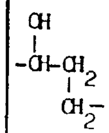 | 4-OH | 2 | 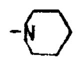 | Base | $C_{24}H_{31}Cl_2NO_5$ | 484,41 | 146 | ANALYSE ELEMENTAIRE<br>% : C / H / N<br>Cal. : 59,50 / 6,45 / 2,89<br>Tr. : 58,80 / 6,56 / 2,59 |
| 89 | 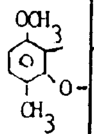 | 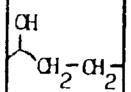 | " | 2 | 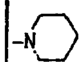 | Base | $C_{24}H_{33}NO_4$ | 399,51 | Huile | RMN($CDCl_3$) δ ppm=entre 6,4 et 7,2,m (6H aromatiques);5,7,s(OH et OH phénolique);5,1,t(H en α de OH);3,98,t (-$OCH_2$);3,75,s($OCH_3$);2,2,s($CH_3$);2,4,m($CH_2$-$CH_2$ et 3 $CH_2$ en α de l'azote); 1,5,m (6 H pipéridiniques). |
| 90 | 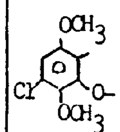 | 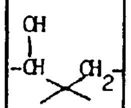 | " | " | " | " | $C_{26}H_{36}ClNO_5$ | 478,01 | 162 | ANALYSE ELEMENTAIRE<br>% : C / H / N<br>Cal. : 65,32 / 7,59 / 2,93<br>Tr. : 64,77 / 5,62 / 2,68 |
| 91 | 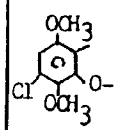 | 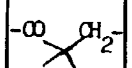 | " | " | " | " | $C_{26}H_{34}ClNO_5$ | 475,99 | 140 | ANALYSE ELEMENTAIRE<br>% : C / H / N<br>Cal. : 65,60 / 7,20 / 2,94<br>Tr. : 65,08 / 7,24 / 2,67 |

TABLEAU I (Suite)

| Numéro de Code | 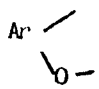 | - A - | $(R)_n$ | m | 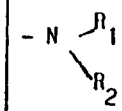 | Forme | Formule brute | Poids moléculaire | Point de fusion (° C) | ANALYSE ELEMENTAIRE OU SPECTRE RMN | | | |
|---|---|---|---|---|---|---|---|---|---|---|---|---|---|
| | | | | | | | | | | ANALYSE ELEMENTAIRE | | | |
| | | | | | | | | | | % | C | H | N |
| 92 | 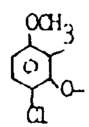 | 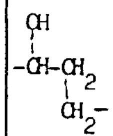 | 4-OH | 2 | 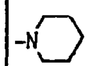 | Base | $C_{23}H_{30}ClNO_4$ | 419,93 | 126 | Cal. | 65,78 | 7,20 | 3,34 |
| | | | | | | | | | | Tr. | 65,77 | 7,33 | 3,33 |
| | | | | | | | | | | | | | |
| | | | | | | | | | | | | | |
| | | | | | | | | | | | | | |

Les dérivés de formule (I) et leurs sels pharmaceutiquement acceptables sont décrits dans les brevets

américains No. 4 536 500 et No. 4 732 896 et dans le brevet belge No. 895 464, brevets qui contiennent toutes informations utiles quant aux procédés de préparation desdits dérivés et sels, à leurs propriétés physico-chimiques et à leur toxicité.

Ces dérivés et sels sont connus d'après ces brevets, comme possédant une activité antagoniste du calcium mise en évidence par le test de la dépolarisation des artères coronaires isolées de chien, ce test ayant permis de conclure que lesdits dérivés et sels trouvaient leur utilité dans le traitement des troubles du système cardiovasculaire.

Il a maintenant été mis en évidence que, de manière inattendue, les dérivés de formule (I) et leurs sels possèdaient en outre une activité antagoniste du calcium au niveau cérébral. Une telle activité a été mise en évidence par le test des contractions de l'artère basilaire isolée de lapin en milieu hyperpotassique selon K. Towart, S. Kazda dans Nimodipine Pharmacological Properties édité par P.E. Betz, K. Deck et F. Hoffmeister (1984), Ed. : E.K. Schattauer Verlag.

Le protocole de ce test est le suivant. Le lapin est anesthésié au pentobarbital à la dose de 30 mg/kg puis sacrifié par exsanguination et décapité au niveau de la 3ème vertèbre cervicale. L'artère basilaire est prélevée (1,5 cm) entre les artères vertébrales et le polygone de Willis. L'artère basilaire est ensuite découpée en spirale sous un angle de 45°. Elle est fixée à l'aide d'une aiguille courbe entre un support en verre en forme de L et un capteur de déplacement (Grass, type FT03). une tension mécanique de 500 mg est appliquée au vaisseau avant de le laisser atteindre son état d'équilibre.

Pendant une période d'une heure, le vaisseau est rincé toutes les 20 minutes, jusqu'à obtention d'un niveau stable.

La contraction tonique consécutive à la dépolarisation provoquée par KC1 est dépendante des mouvements calciques car elle est abolie par la suppression du calcium dans le milieu de survie.

On mesure les concentrations $CI_{50}$ (en M/l) qui réduisent de 50 % l'amplitude de la contraction induite par le KCl à 35 mmoles.

Les résultats obtenus avec certains dérivés de formule (I) dans le test ci-dessus sont rassemblés, à titre d'exemple, dans le Tableau II ci-après.

TABLEAU II

| Numéro de code du dérivé testé | $CI_{50}$ (M/l) |
|---|---|
| 3 | $4.10^{-7}$ |
| 10 | $2,6.10^{-8}$ |
| 69 | $2,3.10^{-9}$ |
| 70 | $7,2.10^{-9}$ |
| 71 | $9,8.10^{-9}$ |
| 72 | $3.10^{-9}$ |
| 74 | $1,4.10^{-9}$ |

Par conséquent, un premier objet de la présente invention réside dans l'utilisation des dérivés de formule (I) et de leurs sels pharmaceutiquement acceptables en thérapeutique, humaine ou animale, pour la préparation d'un médicament pour le traitement des troubles du système cérébrovasculaire, tels que les hémorragies cérébrales, l'infarctus cérébral et la migraine.

Un autre objet de la présente invention réside dans l'utilisation des dérivés de formule (I) et de leurs sels pharmaceutiquement acceptables pour la préparation de compositions pharmaceutiques qui comprennent au moins l'un de ces dérivés et sels en association avec un support ou un excipient physiologiquement acceptable approprié pour lesdits composés, pour le traitement de troubles cérébrovasculaires.

Ces compositions peuvent par exemple être formulées en vue de leur administration par voie orale, pa-

rentérale ou rectale.

Pour l'administration par voie orale, lesdites compositions peuvent prendre la forme de comprimés, dragées ou gélules préparés par les techniques habituelles utilisant des supports et excipients connus tels que des diluants (par exemple, polyéthylène glycol, lactose, dextrose), des liants (par exemple, amidon, gomme arabique, gélatine, méthycellulose, carboxyméthylcellulose, polyvinyl pyrrolidone), des charges, des lubrifiants (par exemple, stéarate de magnésium ou de calcium, acide stéarique, talc, silice) et des agents de désintégration (par exemple, amidon, acide alginique, alginate). Ces compositions, pour administration par voie orale, peuvent également prendre la forme de solutions, de sirops ou de suspensions ; les sirops peuvent contenir, à titre de support, par exemple du saccharose avec éventuellement de la glycérine et/ou du mannitol et/ou du sorbitol ; les suspensions peuvent contenir, à titre de support, par exemple une gomme naturelle, de l'agar, de l'alginate de sodium, de la pectine ou de la méthylcellulose ; quant aux solutions, il pourra s'agir de solutions aqueuses.

Pour l'administration par voie parentérale, les compositions selon l'invention peuvent prendre la forme de solutions, suspensions ou émulsions injectables comprenant un véhicule liquide, huileux ou aqueux, parentéralement acceptable (par exemple, eau stérile, huile d'olive, oléate d'éthyle, propylène glycol dans le cas des formes pour l'injection intramusculaire et eau stérile ou solutions aqueuses salines isotoniques stériles dans le cas des formes pour injection intraveineuse).

Enfin pour l'administration par voie rectale, les compositions selon l'invention peuvent prendre la forme de suppositoires comprenant les bases habituelles pour suppositoires (par exemple, beurre de cacao, polyéthylène glycol).

La dose à laquelle les principes actifs, c'est-à-dire les dérivés de formule (I) et leurs sels, peuvent être administrés à l'homme ou à tout animal à sang chaud, dépend notamment de la voie d'administration, du poids corporel et de l'état pathologique du patient et de la puissance thérapeutique des dérivés et sels mis en oeuvre. Généralement, par voie orale, les doses pourront atteindre 500 mg de principe actif par jour (en une ou plusieurs prises) ; par voie parentérale, les doses pourront atteindre 100 mg de principe actif par jour (en une ou plusieurs injections journalières) et par voie rectale, les doses pourront atteindre 300 mg de principe actif par jour (en un ou plusieurs suppositoires).

Dans un but d'illustration de la présente invention, on donne ci-après un certain nombre d'exemples de compositions pharmaceutiques réalisées par mise en oeuvre des composés de formule (I) :

Exemple 1 : Préparation de comprimés.

On mélange intimement entre eux les constituants suivants :

- 100 mg de composé de numéro de code 3
- 20 mg de lactose
- 4 mg de carboxyméthylamidon sodique
- 4 mg de polyvinylpyrrolidone
- 2 mg de stéarate de magnésium.

On fabrique avec ce mélange des comprimés sécables (munis de deux barrettes de sécabilité) pesant chacun 130 mg.

Exemple 2 : Préparation de comprimés avec les constituants suivants :

- 10 mg de composé de numéro de code 10
- 100 mg de lactose
- 8 mg d'amidon de maïs
- 8 mg de polyvinylpyrrolidone
- 4 mg de stéarate de magnésium.

On fabrique en opérant comme dans l'exemple prédédent, des comprimés d'un poids final de 130 mg.

Exemple 3 : Préparation d'une solution pour administration orale.

La préparation d'une telle solution contenant 10 mg de principe actif pour 5 ml de solution est réalisée en dissolvant :

- 0,2 g de composé de numéro de code 69
- 0,1 g de parahydroxybenzoate de méthyle
- 0,1 g de parahydroxybenzoate de propyle
- q.s.p. 100 ml d'eau purifiée.

Exemple 4 : Préparation d'un sirop.

La préparation d'un sirop contenant 100 mg de principe actif pour 5 ml de sirop est réalisée en mélangeant :

- 2 g de composé de numéro de code 13
- 40 g de saccharose
- 10 g de glycérine
- édulcorant et aromatisant
- q.s.p. 100 ml d'eau purifiée.

## Revendications

**1.** Utilisation des dérivés aminoalkoxy aromatiques répondant à la formule générale :

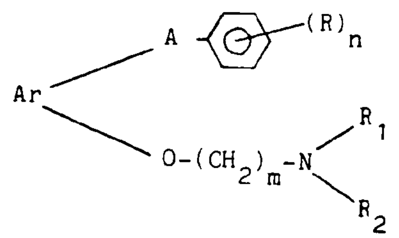

(I)

dans laquelle :

- R représente un atome d'hydrogène ou d'halogène, un groupe méthyle, hydroxyle, alkoxy où le reste alkyle comporte de 1 à 4 atomes de carbone, ou benzyloxy ;
- n prend la valeur 1 ou 2 quand R est différent de H ;
- m prend la valeur 2 ou 3 ;
- A représente un enchaînement ayant l'une quelconque des structures suivantes :

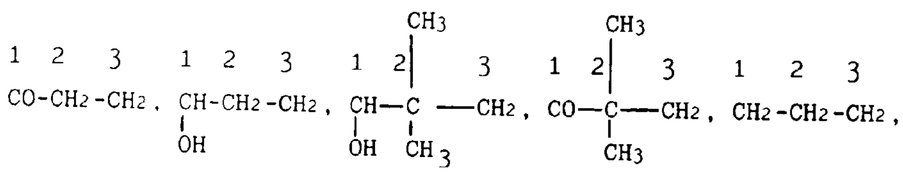

le groupe aromatique Ar étant relié à la position 1 de cet enchaînement,

- le couple ($R_1$, $R_2$) prend la valeur (H, alkyle en $C_1$-$C_4$), (H, cycloalkyle en $C_5$-$C_6$) ou (H, cycloalkylalkyle comportant de 4 à 8 atomes de carbone), excepté dans le cas où

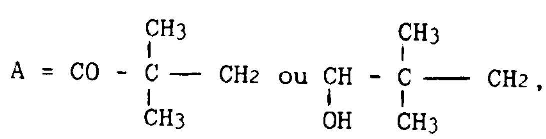

ou encore la valeur (alkyle en $C_1$-$C_4$, alkyle en $C_1$-$C_4$), $R_1$ et $R_2$ pouvant également former conjointement avec l'atome d'azote auquel ils sont liés, un radical choisi parmi les suivants : pyrrolidino, pipéridino, hexaméthylèneimino, morpholino ; et
- Ar représente :
  . soit un groupe benzènique de structure:

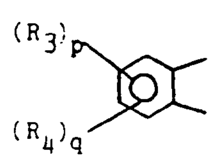

dans lequel $R_3$ représente un atome d'halogène ou un groupe nitro ou méthyle, $R_4$ = alkoxy de 1 à 4 atomes de carbone, p = 0, 1 ou 2, q = 0,1,2,3,4, $p + q \leqq 4$, avec les restrictions que lorsque

$$A = CO-CH_2-CH_2 \text{ ou } \underset{\substack{|\\OH}}{CH}-CH_2-CH_2,$$

p et q ne peuvent prendre simultanément la valeur 0,

. soit un groupe naphtalènique ou benzodioxannique respectivement de structure :

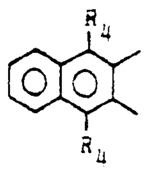

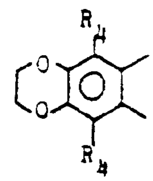

où $R_4$ a les mêmes significations que précédemment,

ainsi que des sels d'addition d'acide minéral ou organique pharmaceutiquement acceptables, pour la fabrication d'un médicament pour le traitement des troubles du système cérébrovasculaire.

2. Utilisation selon la revendication 1, caractérisée en ce que les troubles du système cérébrovasculaire comprennent les hémorragies cérébrales, l'infarctus cérébral et la migraine.

3. Utilisation selon la revendication 1 ou 2, de dérivés et sels pour lesquels A représente l'enchaînement de structure

$$\overset{1}{CO}-\overset{2}{CH_2}-\overset{3}{CH_2}.$$

4. Utilisation selon la revendication 3, de dérivés et sels pour lesquels l'ensemble

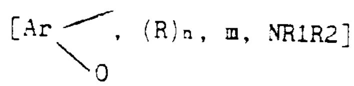

prend la valeur :

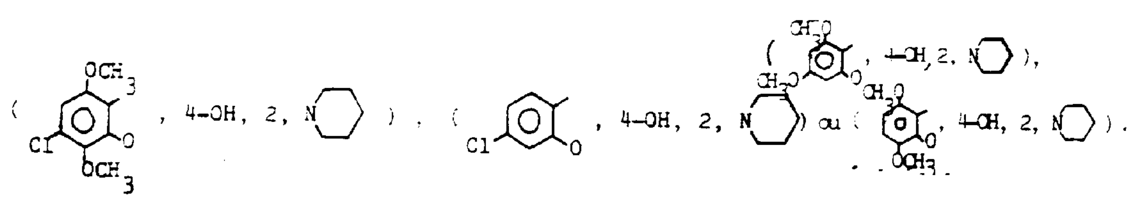

( , 4-OH, 2, ) , ( , 4-OH, 2, ) ou ( , 4-OH, 2, ), ( , 4-OH, 2, ) .

5. Utilisation selon la revendication 1 ou 2, de dérivés et sels pour lesquels A représente l'enchaînement de structure:

$$\overset{1}{CO} - \overset{2}{\underset{\substack{|\\CH_3}}{\overset{\substack{CH_3\\|}}{C}}} --- \overset{3}{CH_2}$$

6. Utilisation selon la revendication 5, de dérivés et sels pour lesquels l'ensemble

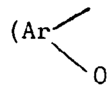 , $(R)_n$, m, NR1R2)

prend l'une quelconque des valeurs suivantes :

( 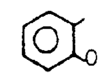 , 4-$OCH_2$Ø, 2, 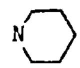 ), ( 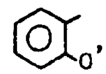 , 4-OH, 2, 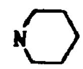 ),

( 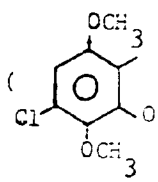 , 4-OH, 2, 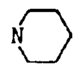 ).

7. Utilisation selon la revendication 1 ou 2, de dérivés et sels pour lesquels A représente l'enchaînement de structure $CH_2$-$CH_2$-$CH_2$.

8. Utilisation selon la revendication 7, de dérivés et sels pour lesquels l'ensemble

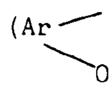 , $(R)_n$, m, NR1R2)

prend l'une quelconque des valeurs suivantes :

( 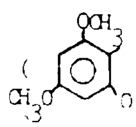 , 4-OH, 2, 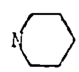 ), ( 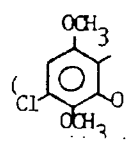 , 4-OH, 2, 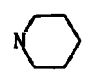 ).

9. Utilisation selon la revendication 1 ou 2 de dérivés et sels pour lesquels A représente l'enchaînement

$$\overset{\overset{OH}{|}}{C}H-CH_2-CH_2 .$$

10. Utilisation selon la revendication 9, de dérivés et sels pour lesquels l'ensemble

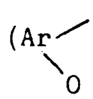 , $(R)_n$, m, NR1R2)

prend l'une quelconque des valeurs suivantes :

( 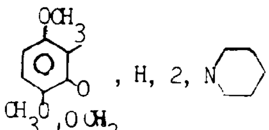 , H, 2, ) ; ( 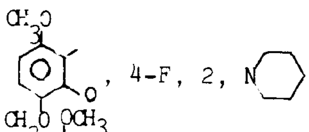 , 4-F, 2, )

( 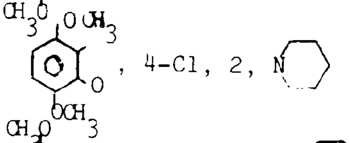 , 4-Cl, 2, ) ; ( 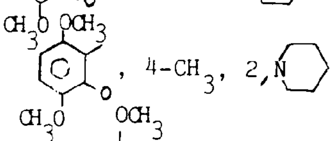 , 4-$CH_3$, 2, ) ;

( 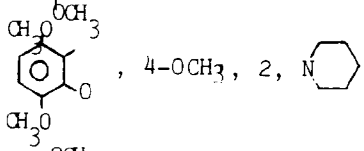 , 4-$OCH_3$, 2, ) ; ( 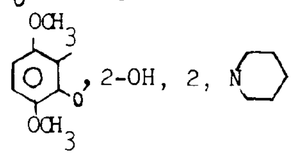 , 2-OH, 2, ) ;

( 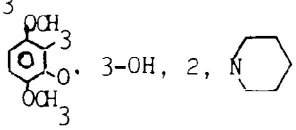 , 3-OH, 2, ) ; ( 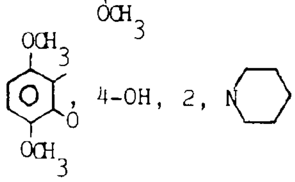 , 4-OH, 2, ) ;

( 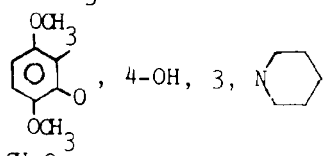 , 4-OH, 3, ) ; ( 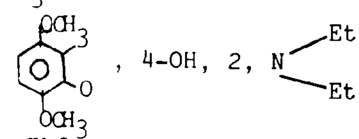 , 4-OH, 2, ) ;

( 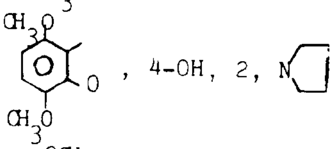 , 4-OH, 2, ) ; ( 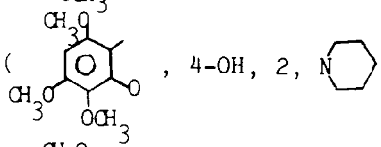 , 4-OH, 2, ) ;

( 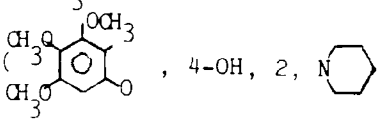 , 4-OH, 2, ) ; ( 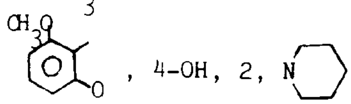 , 4-OH, 2, ) ;

( 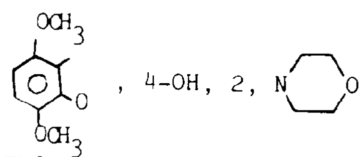 , 4-OH, 2, ) ; ( 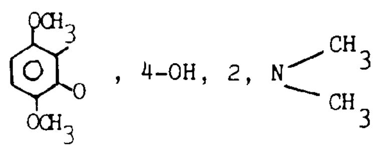 , 4-OH, 2, ) ;

( 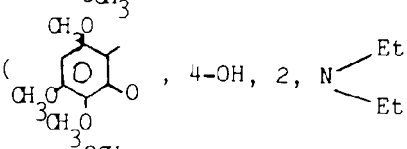 , 4-OH, 2, ) ; ( 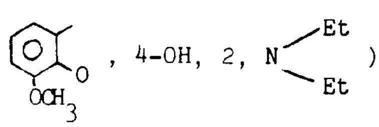 , 4-OH, 2, ) ;

( 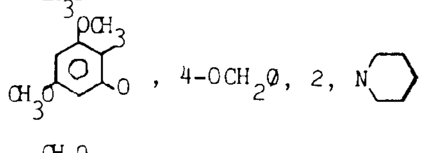 , 4-$OCH_2Ø$, 2, ) ; ( 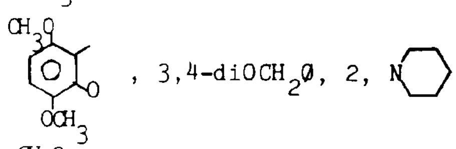 , 3,4-di$OCH_2Ø$, 2, ) ;

( 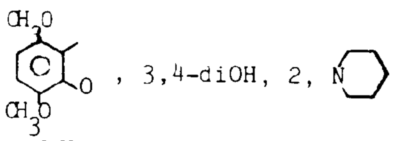 , 3,4-diOH, 2, ) ; ( 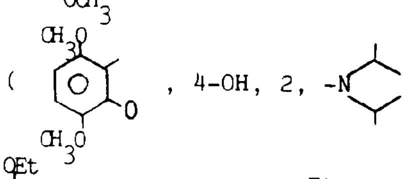 , 4-OH, 2, - ) ;

( 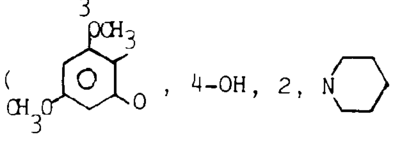 , 4-OH, 2, ) ; ( 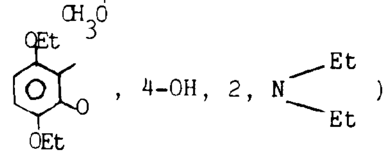 , 4-OH, 2, ) ;

( 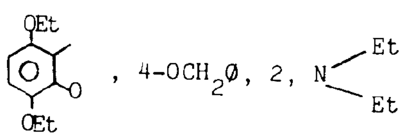 , 4-$OCH_2Ø$, 2, ) ;

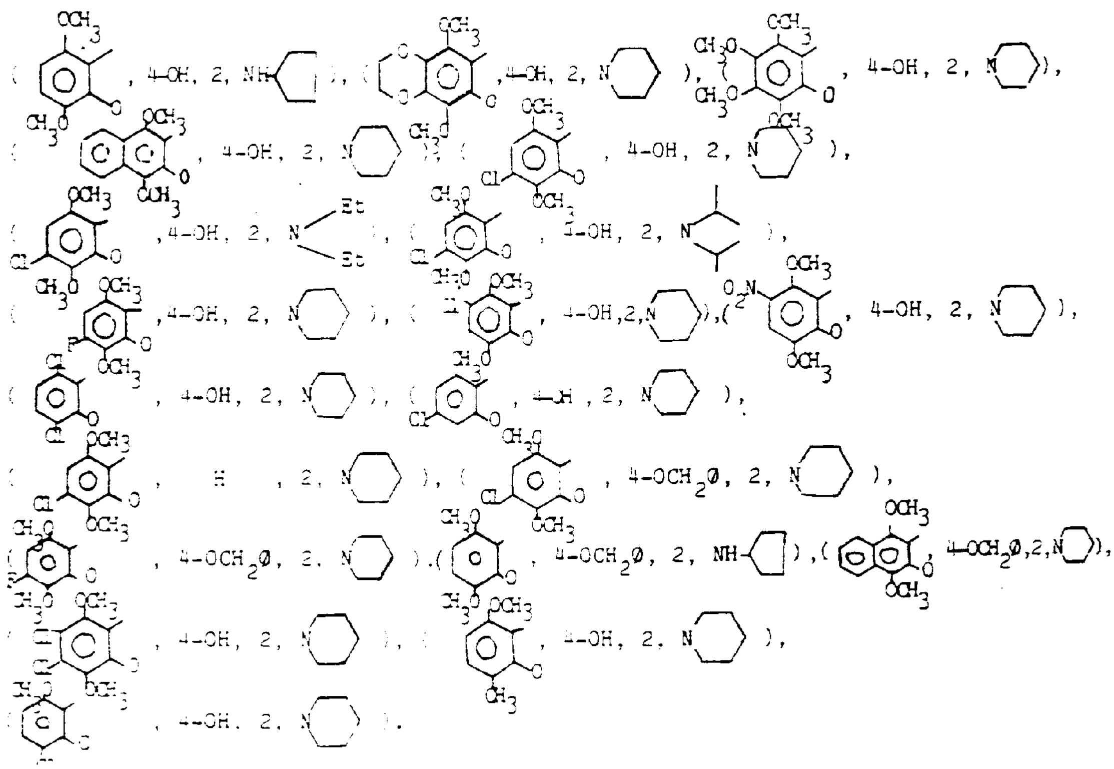

11. Utilisation selon la revendication 1 ou 2 de dérivés et sels pour lesquels A représente l'enchaînement de structure

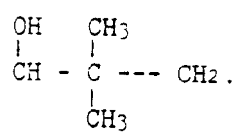

12. Utilisation selon la revendication 11, de dérivés et sels pour lesquels l'ensemble

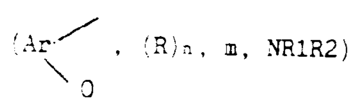

prend la valeur

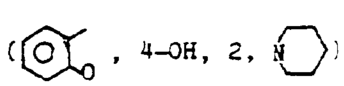

ou

( 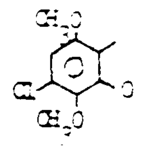 , 4-OH, 2, 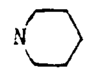 ).

## Patentansprüche

1. Verwendung von Aminoalkoxyderivaten von Aromaten entsprechend der allgemeinen Formel:

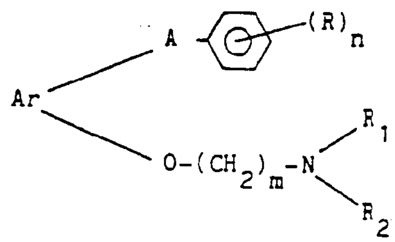

(I)

in der:

- R ein Wasserstoff- oder Halogenatom, eine Methyl-, Hydroxyl-, oder Alkoxygruppe, in der der Alkylrest 1 bis 4 Kohlenstoffatome umfaßt, oder eine Benzyloxygruppe darstellt;
- n den Wert 1 oder 2 hat, wenn R von H verschieden ist;
- m den Wert 2 oder 3 hat,
- A eine Kette mit einer der folgenden Strukturen darstellt:

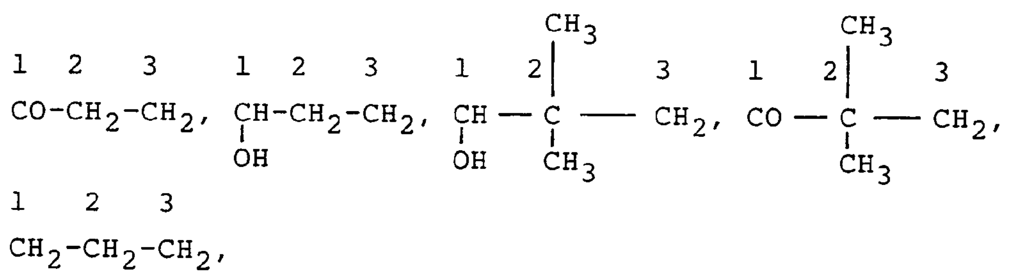

wobei die aromatische Gruppe Ar mit der Position 1 dieser Kette verbunden ist,

- das Paar ($R_1$, $R_2$) die Bedeutung (H, Alkyl mit $C_1$-$C_4$), (H, Cycloalkyl mit $C_5$-$C_6$) oder (H, Cycloalkylalkyl mit 4 bis 8 Kohlenstoffatomen), mit Ausnahme des Falles, in dem

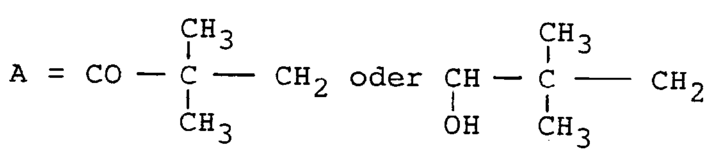

bedeutet;

oder auch noch die Bedeutung (Alkyl mit $C_1$-$C_4$, Alkyl mit $C_1$-$C_4$) annimmt, wobei $R_1$ und $R_2$ auch gemeinsam mit dem Stickstoffatom, an welches sie gebunden sind, eine Gruppe bilden können, die aus den folgenden ausgewählt ist: Pyrrolidin, Piperidin, Hexamethylenimin, Morpholin; und

- Ar darstellt:

  entweder eine Benzolgruppe der Struktur:

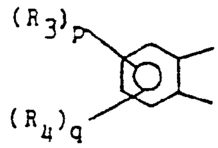

in der $R_3$ ein Halogenatom oder eine Nitro- oder Methylgruppe, $R_4$ = Alkoxy von 1 bis 4 Kohlenstoffatomen, p = 0, 1 oder 2, q = 0,1,2,3,4, p + q ≦ 4, mit der Einschränkung, daß p und q nicht gleichzeitig den Wert 0 annehmen können, wenn A = $CO-CH_2-CH_2$ oder

$$\underset{\displaystyle OH}{CH}-CH_2-CH_2$$

ist,

oder eine Naphtalin- oder Benzodioxangruppe der Struktur:

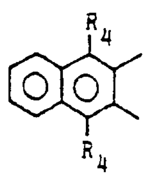

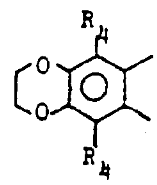

wo $R_4$ die gleiche Bedeutung wie vorhergehend hat, so wie Additionssalze einer Mineralsäure- oder organischen Säure, die pharmazeutisch akzeptabel sind, für die Herstellung eines Medikaments zur Behandlung zerebrovaskulärer Störungen.

2. Verwendung nach Anspruch 1, dadurch gekennzeichnet, daß die zerebrovaskulären Störungen Hirnblutungen, Zerebralinfarkt und Migräne umfaßt.

3. Verwendung nach Anspruch 1 oder 2, von Derivaten und Salzen, in denen A eine Kette der Struktur

$$\overset{1}{CO}-\overset{2}{CH_2}-\overset{3}{CH_2}$$

darstellt.

4. Verwendung nach Anspruch 3, von Derivaten und Salzen, in denen die Gruppe

[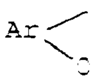, (R)n, m, NR1R2]

die folgende Bedeutung hat:

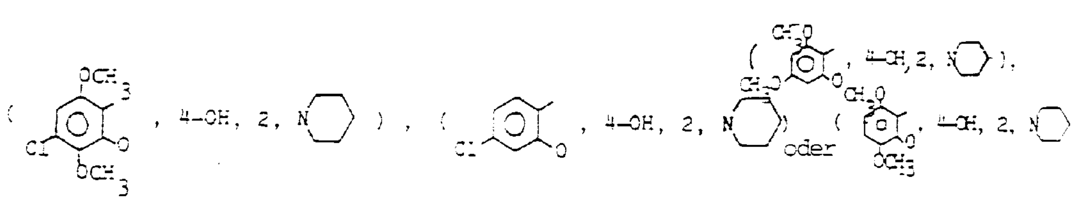

( , 4-OH, 2, ), ( , 4-OH, 2, ), ( , 4-OH, 2, ), oder ( , 4-OH, 2, )

5. Verwendung nach Anspruch 1 oder 2, von Derivaten und Salzen, in denen A eine Kette der Struktur

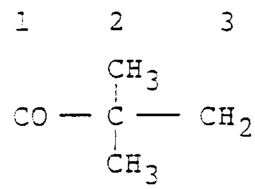

darstellt.

**6.** Verwendung nach Anspruch 5, von Derivaten und Salzen, in denen die Gruppe

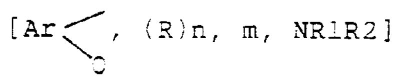

eine der folgenden Bedeutungen hat:

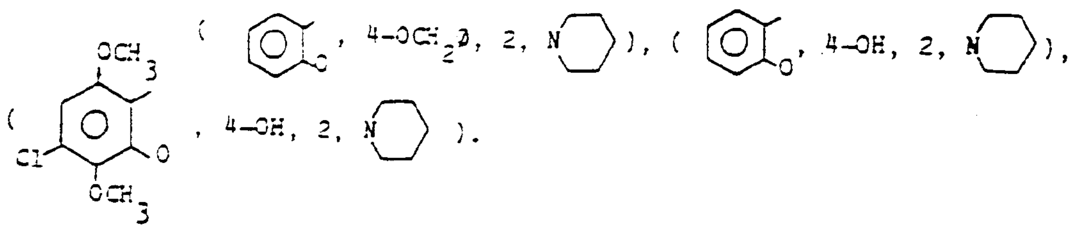

**7.** Verwendung nach Anspruch 1 oder 2, von Derivaten und Salzen, in denen A eine Kette der Struktur $CH_2$-$CH_2$-$CH_2$ darstellt.

**8.** Verwendung nach Anspruch 7, von Derivaten und Salzen, in denen die Gruppe

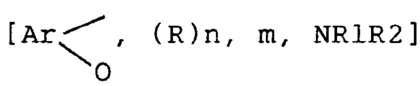

eine der folgenden Bedeutungen hat:

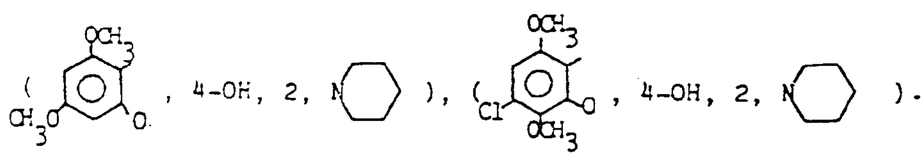

**9.** Verwendung nach Anspruch 1 oder 2, von Derivaten und Salzen, in denen A eine Kette der Struktur

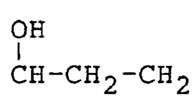

darstellt.

**10.** Verwendung nach Anspruch 9, von Derivaten und Salzen, in denen die Gruppe

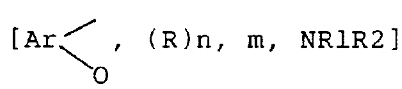

eine der folgenden Bedeutungen hat:

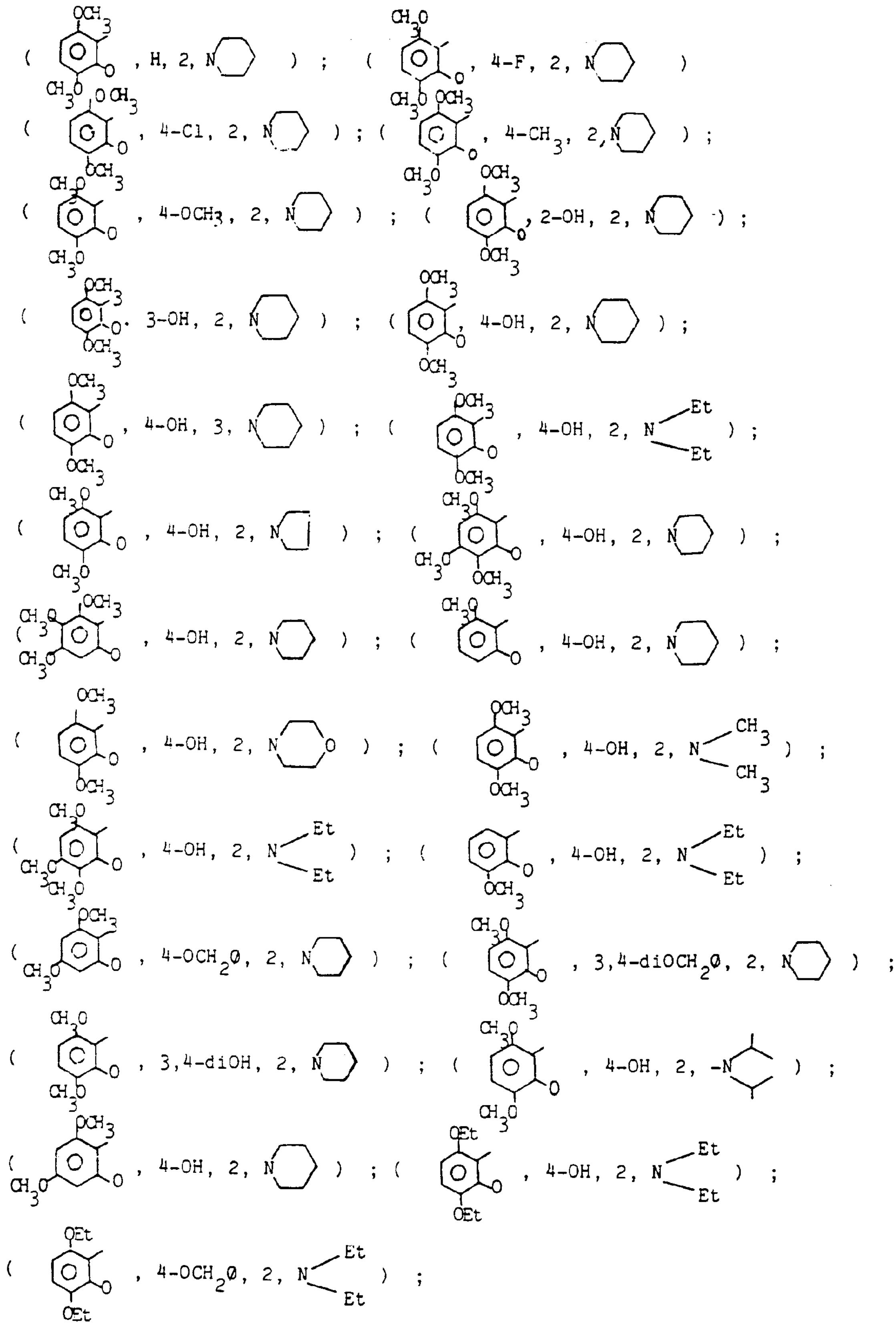

( , H, 2, ) ; ( , 4-F, 2, )

( , 4-Cl, 2, ) ; ( , 4-$CH_3$, 2, ) ;

( , 4-$OCH_3$, 2, ) ; ( , 2-OH, 2, ) ;

( , 3-OH, 2, ) ; ( , 4-OH, 2, ) ;

( , 4-OH, 3, ) ; ( , 4-OH, 2, ) ;

( , 4-OH, 2, ) ; ( , 4-OH, 2, ) ;

( , 4-OH, 2, ) ; ( , 4-OH, 2, ) ;

( , 4-OH, 2, ) ; ( , 4-OH, 2, ) ;

( , 4-OH, 2, ) ; ( , 4-OH, 2, ) ;

( , 4-$OCH_2$Ø, 2, ) ; ( , 3,4-di$OCH_2$Ø, 2, ) ;

( , 3,4-diOH, 2, ) ; ( , 4-OH, 2, ) ;

( , 4-OH, 2, ) ; ( , 4-OH, 2, ) ;

( , 4-$OCH_2$Ø, 2, ) ;

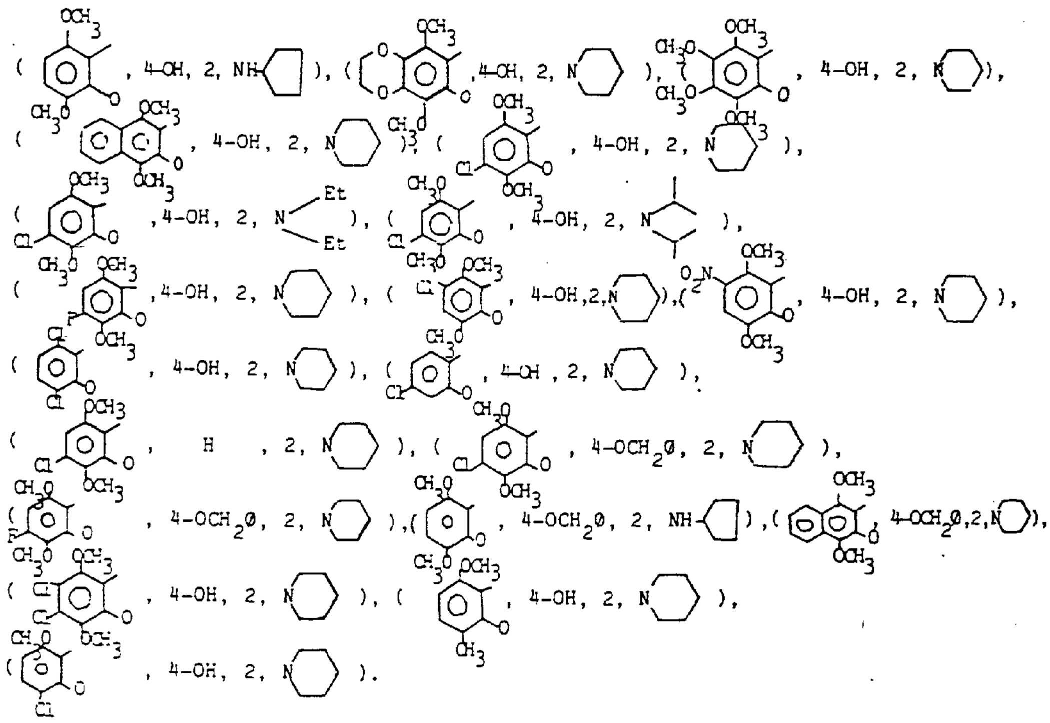

11. Verwendung nach Anspruch 1 oder 2, von Derivaten und Salzen, in denen A eine Kette der Struktur

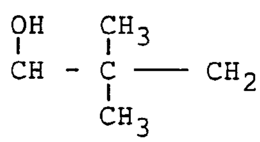

darstellt.

12. Verwendung nach Anspruch 11, von Derivaten und Salzen, in denen die Gruppe

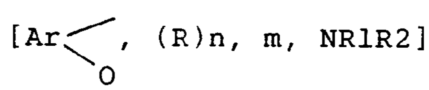

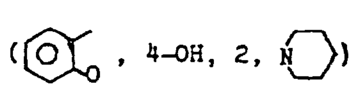

oder

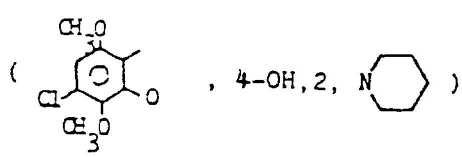

bedeutet.

## Claims

1. Use of the aromatic amino-alkoxy derivatives of formula (I) :

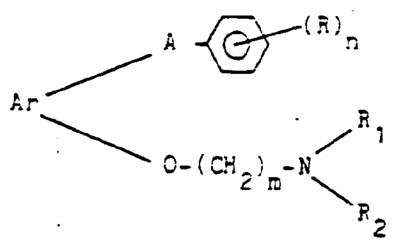

(I)

in which :

- R represents a hydrogen or halogen atom or a methyl, hydroxyl, $C_1$-$C_4$ alkoxy or benzyloxy group ;
- n takes the value 1 or 2 when R is different from H ;
- m takes the value 2 or 3 ;
- A represents a chain having any of the following structures :

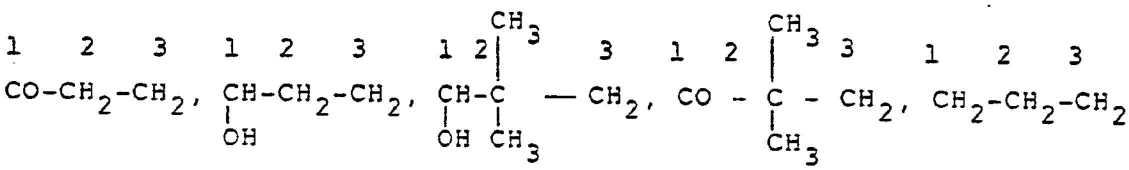

the aromatic group Ar being bonded to position 1 of this chain,
- the pair ($R_1$,$R_2$) takes the value (H, $C_1$-$C_4$ alkyl), (H, $C_5$-$C_6$ cycloalkyl) or (H, cycloalkylalkyl comprising 4 to 8 carbon atoms),
except in the case where

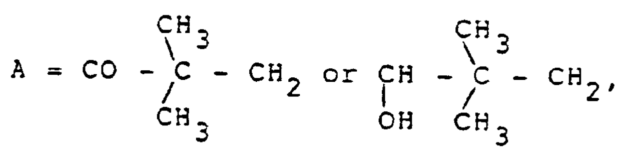

or else the value ($C_1$-$C_4$ alkyl, $C_1$-$C_4$ alkyl), $R_1$ and $R_2$ being also able to form jointly with the nitrogen atom with which they are bonded, a radical chosen from the following : pyrrolidino, piperidino, hexamethyleneimino, morpholino ; and
- Ar represents :
  . either a benzenic group of structure :

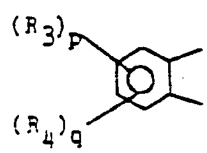

in which $R_3$ represents a halogen atom or a nitro or methyl group, $R_4$ = alkoxy with 1 to 4 carbon atoms, p = 0, 1 or 2, q = 0, 1, 2, 3 or 4, p + q $\leqq$4, with the restrictions that when
A = $CO$-$CH_2$-$CH_2$ or

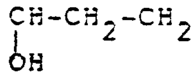

p and q cannot take simultaneously the value 0,
. or a naphtalenic or benzodioxannic group respectively of structure :

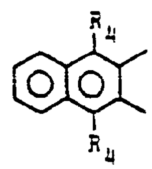

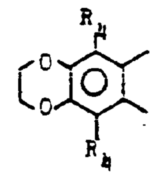

where $R_4$ has the same meanings as above,
as well as of their pharmaceutically acceptable inorganic or organic acid addition salts, for the manufacture of a drug for the treatment of the troubles of the cerebrovascular system.

**2.** The use according to claim 1, wherein said troubles of the cerebrovascular system comprise cerebral haemorrhages, cerebral infraction and migraine.

**3.** The use according to claim 1 or 2, of derivatives and salts wherein A represents the chain of structure

$$\overset{1}{CO} - \overset{2}{CH_2} - \overset{3}{CH_2}.$$

**4.** The use according to claim 3, of derivatives and salts wherein

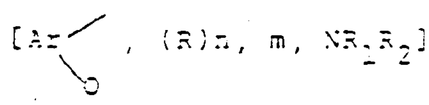

takes the value

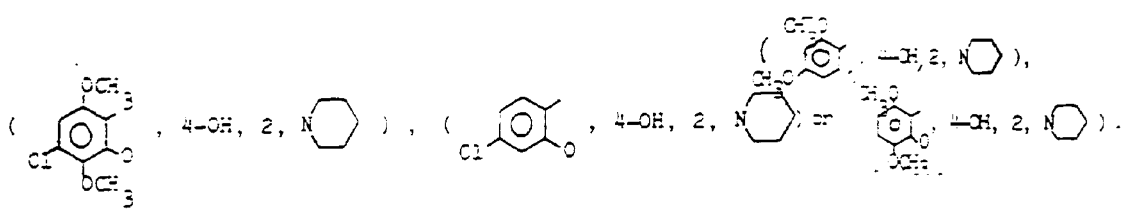

**5.** The use according to claim 1 or 2, of derivatives and salts wherein A represents the chain of structure :

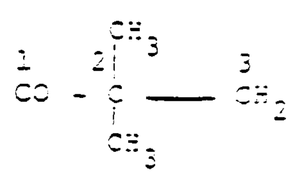

**6.** The use according to claim 5, of derivatives and salts wherein

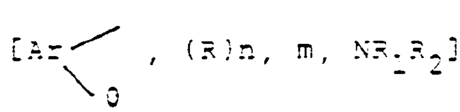

takes any one of the following values :

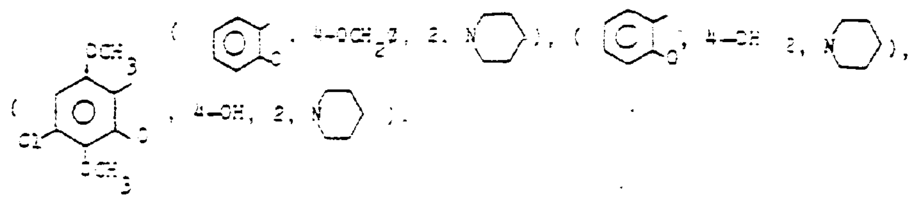

**7.** The use according to claim 1 or 2, of derivatives and salts wherein A represents the chain of structure

$CH_2 - CH_2 - CH_2$

**8.** The use according to claim 7, of derivatives and salts wherein

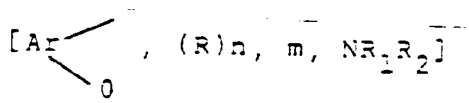

takes any one of the following values :

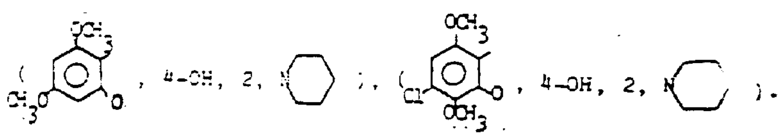

**9.** The use according to claim 1 or 2, of derivatives and salts wherein A represents the chain

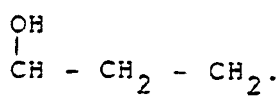

**10.** The use according to claim 9, of derivatives and salts wherein

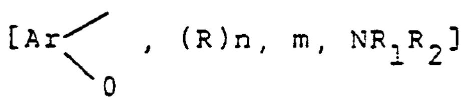

takes any one of the following values :

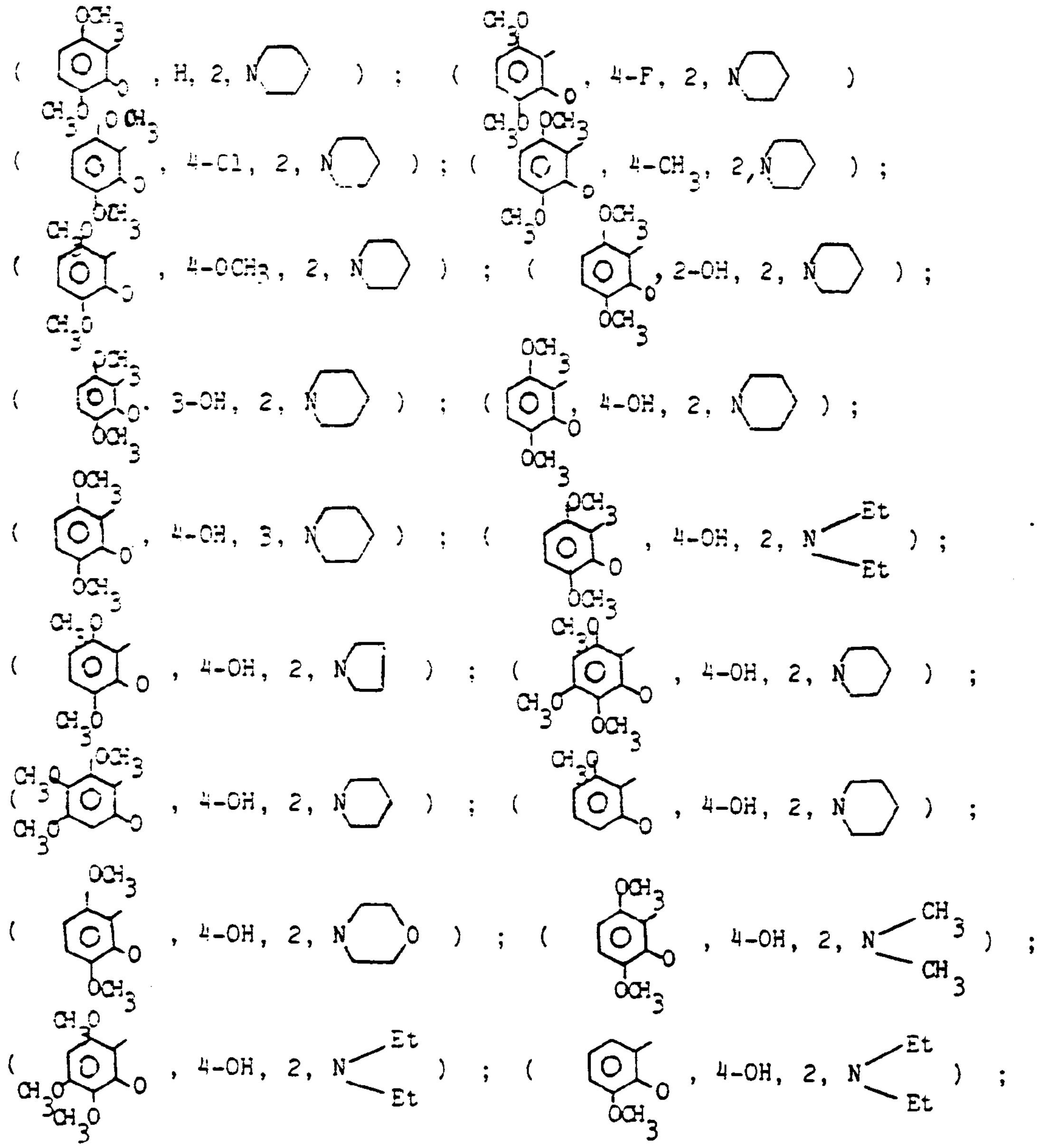

( , H, 2, ) ; ( , 4-F, 2, )

( , 4-Cl, 2, ) ; ( , 4-$CH_3$, 2, ) ;

( , 4-$OCH_3$, 2, ) ; ( , 2-OH, 2, ) ;

( , 3-OH, 2, ) ; ( , 4-OH, 2, ) ;

( , 4-OH, 3, ) ; ( , 4-OH, 2, ) ;

( , 4-OH, 2, ) ; ( , 4-OH, 2, ) ;

( , 4-OH, 2, ) ; ( , 4-OH, 2, ) ;

( , 4-OH, 2, ) ; ( , 4-OH, 2, ) ;

( , 4-OH, 2, ) ; ( , 4-OH, 2, ) ;

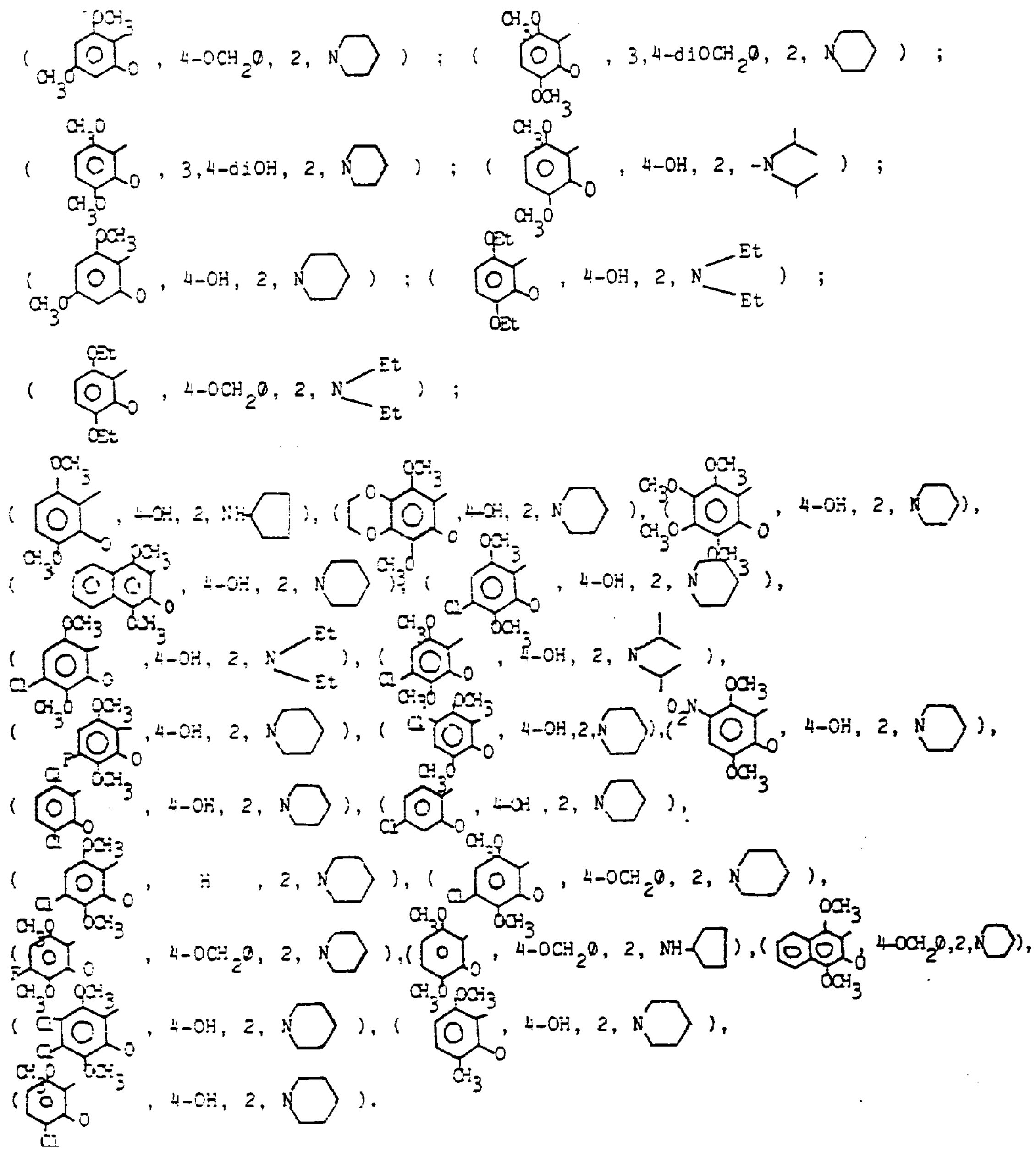
( , 4-$OCH_2$Ø, 2, ) ; ( , 3,4-di$OCH_2$Ø, 2, ) ;
( , 3,4-diOH, 2, ) ; ( , 4-OH, 2, ) ;
( , 4-OH, 2, ) ; ( , 4-OH, 2, ) ;
( , 4-$OCH_2$Ø, 2, ) ;
( , 4-OH, 2, ), ( , 4-OH, 2, ), ( , 4-OH, 2, ),
( , 4-OH, 2, ), ( , 4-OH, 2, ),
( , 4-OH, 2, ), ( , 4-OH, 2, ),
( , 4-OH, 2, ), ( , 4-OH,2, ), ( , 4-OH, 2, ),
( , 4-OH, 2, ), ( , 4-OH , 2, ),
( , H , 2, ), ( , 4-$OCH_2$Ø, 2, ),
( , 4-$OCH_2$Ø, 2, ), ( , 4-$OCH_2$Ø, 2, ), ( , 4-$OCH_2$Ø,2, ),
( , 4-OH, 2, ), ( , 4-OH, 2, ),
( , 4-OH, 2, ).

**11.** The use according to claim 1 or 2, of derivatives and salts wherein A represents the chain of structure :

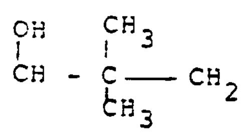

**12.** The use according to claim 11, of derivatives and salts wherein

[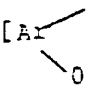 , $(R)_n$, m, $NR_1R_2$]

takes the value :

(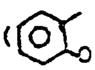 , 4-OH, 2, 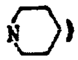) or ( 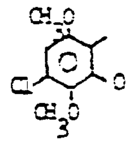 , 4-OH, 2, 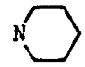 ).